(19) Europäisches Patentamt
European Patent Office
Office européen des brevets

(11) EP 3 628 659 A1

(12) **EUROPEAN PATENT APPLICATION**

(43) Date of publication:
**01.04.2020 Bulletin 2020/14**

(21) Application number: **18382693.2**

(22) Date of filing: **28.09.2018**

(51) Int Cl.:
*C07C 391/00* (2006.01) *A61K 31/095* (2006.01)
*A61K 31/12* (2006.01) *A61K 31/277* (2006.01)
*A61P 31/04* (2006.01) *A61P 35/00* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Consejo Superior De Investigaciones Científicas 28006 Madrid (ES)**
- **Universidad de Navarra 31009 Pamplona (Navarra) (ES)**
- **University of Szeged 6720 Szeged (HU)**
- **Universität des Saarlandes 66123 Saarbrücken (DE)**

(72) Inventors:
- **DOMÍNGUEZ ÁLVAREZ, Enrique 28006 Madrid (ES)**
- **SPENGLER, Gabriella 6720 Szeged (HU)**
- **JACOB, Claus 66041 Saarbrücken (DE)**
- **SANMARTÍN GRIJALBA, María del Carmen 31008 Pamplona (Navarra) (ES)**

(74) Representative: **Pons Glorieta Ruben Dario 4 28010 Madrid (ES)**

(54) **SELENOESTER-CONTAINING COMPOUNDS FOR USE IN THE TREATMENT OF MICROBIAL INFECTIONS OR COLORECTAL CANCER**

(57) The invention relates to a compound of formula I for use in the treatment of microbial infections or colorectal cancer, wherein $R_1$ to $R_4$, X, n, p, q, r and s are defined in the description.

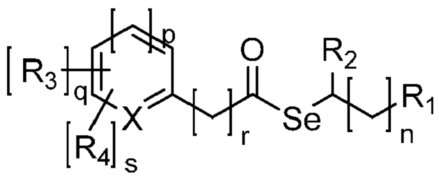

I

EP 3 628 659 A1

## Description

The invention relates to a compound of formula **I** for use in the treatment of microbial infections or colorectal cancer.

## BACKGROUND ART

According to the Globocan 2012 report of the IARC, the world risk of getting cancer before age 75 is of 18.5%, and according to the annual reports of United States National Statistic System, cancer is the second leading cause of death in the United States, only behind heart diseases. Colorectal cancer (CRC) is one of the most frequent types of cancer, only behind lung and breast cancer (Globocan 2012) and thus finding novel treatments of this cancer location is crucial for the health systems. Colorectal cancer, also known as bowel cancer and colon cancer, is the development of cancer from the colon or rectum (parts of the large intestine). Treatments used for colorectal cancer may include some combination of surgery, radiation therapy, chemotherapy and targeted therapy. Selenocompounds are emerging as novel approach as many of them showed interesting activity against different cancer cells, including among them colorectal cancer cell lines such as Colo205, Colo320, HCT-116 and HT-29 (Gajdács et al, 2017, Bioorg Med Chem Lett. 27(4): 797-802; Álvarez-Pérez et al, 2018, Molecules, 23(3): E628).

Cancer multidrug resistance is becoming slowly a troublesome problem due to the appearance of resistant types of cancer. In this context, the search for inhibitors of efflux pumps is a promising approach in the fight against resistance (Spengler et al, 2011, Anticancer Res. 31:3285-3288). These efflux pumps are proteins overexpressed by resistant cancer cells which recognize exogenous agents as anticancer drugs to extrude them out of the cell, preventing them to exert their action and thus developing resistance to them. In this context, certain selenium-containing compounds have shown a very promising capacity to inhibit the efflux pumps, showing an activity up to 4-fold higher than the reference compound. Additionally, these compounds showed a potent cytotoxic activity accompanied by a noteworthy selectivity towards cancer cells in respect to non-tumoral cells (Gajdács et al, 2017, Bioorg Med Chem Lett. 27(4): 797-802).

Similarly, resistant bacterial infections are becoming a troublesome and worrying problem nowadays, due to the worldwide increase of the bacterial infections which are resistant to several antibiotics, and that are reducing the efficacy of the antibiotics currently used in clinical practice (Pages et al, 2011, Curr Med Chem, 18:2969-80). Resistant bacterial and fungal infections caused around 27,500 deaths in 2013 in the United States alone ("*Antibiotics Resistance Threats, US 2013*", US Centers for Disease Control and Prevention). The bacterial and fungal strains more frequently involved in resistant infections are *Clostridium difficile,* methicillin resistant *Staphylococcus aureus* (MRSA), *Streptococcus pneumoniae*, *Enterobacteriaceae* (including known bacterial strains as *Escherichia coli*, *Klebsiella pneumonia*, *Enterococcus spp.* and *Salmonella typhimurium*, among others), *Acinetobacter baumanii* and *Candida spp.* All these microbes can cause healthcare-associated infections ("*Antibiotics Resistance Threats*, *US 2013*", US Centers for Disease Control and Prevention). MRSA, for instance, can cause a range of bacterial illnesses, such as skin infections, wound infections, endocarditis, osteomyelitis, severe pneumonia, arthritis and bloodstream infections which can cause sepsis (Dayan et al, 2016, Expert Rev Vaccines, 15:1373-1392). And the fungi *Candida*, especially those strains resistant to fluconazole, can cause candidiasis, a severe nosocomial bloodstream infection (Ben-Ami, 2018, J Fungi, 4:E97).

Within this frame, the discovery of novel effective antibiotics, or of novel inhibitors of the resistance mechanisms as the efflux pumps involved in resistance is crucial in the fight against resistant infections. Certain forms of selenium, such as sodium selenite (Kumar et al, 2010, Scand J Infect Dis, 42:266-274) or selenium nanoparticles (Cihalova et al, 2015, Int J Mol Sci, 16:24656-24672), have shown antimicrobial properties or anti-biofilm properties against resistant bacteria. Knowing the activity of the selenoester-derived compounds against resistant cancer cells (Gajdács et al, 2017, Bioorg Med Chem Lett. 27(4): 797-802), we have tested them against selected bacterial and fungal pathogens with promising results.

Therefore, it would be desirable to have series of novel selenocompounds that include in their structure a selenoester moiety (-CO-Se-) for its use as anticancer agents and as efflux-pump inhibitors for the treatment of microbial infections

## SUMMARY OF THE INVENTION

One aspect of the present invention relates to a compound of formula **I** for use in the treatment of microbial infections or colorectal cancer:

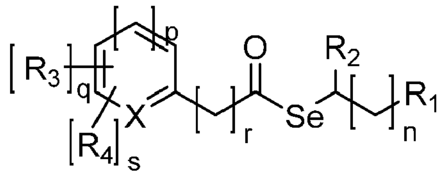

I

wherein:

$R_1$ represents -Ph, -CN, -Ph$R_5$, -CO$R_5$, -S$R_5$, -(SO)$R_5$ or -($SO_2$)$R_5$;
$R_2$ represents -H, -$CH_3$, -$CH_2CH_3$, -Ph or -CO$CH_3$;
each $R_3$ independently represents -$(CH_2)_r$-COSeCH$R_2$$(CH_2)_n$-$R_1$;
each $R_4$ independently represents -H, -F, -Cl, -Br, -I, -$(CH_2)_t$H, -CH$(CH_3)_2$,-C$(CH_3)_3$, -OH, -O$R_6$, -OCO$R_6$, -S$R_6$, -(SO)$R_6$, -($SO_2$)$R_6$, -CN, -$CF_3$, -CO$R_6$, -COOH,-Ph, -$NH_2$, -NH$R_6$, -N$(R_6)_2$, or -$NO_2$;
each $R_5$ independently represents -$(CH_2)_t$H, -CH$(CH_3)_2$, -C$(CH_3)_3$, -$CF_3$, -Ph or-Ph$R_4$;
each $R_6$ independently represents -$(CH_2)_t$H, -CH$(CH_3)_2$, -C$(CH_3)_3$ or-Ph;
t represents an integer that ranges between 1 and 6;
X represents CH, N or S;
p represents 0 or 1;
n represents an integer that ranges between 0 and 5;
q represents 0, 1 or 2;
r represents 0, 1 or 2; and
s represents an integer that ranges between 1 and 3,
with the proviso that the following compounds are excluded:

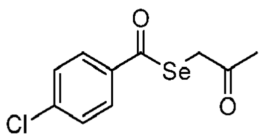

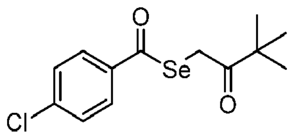

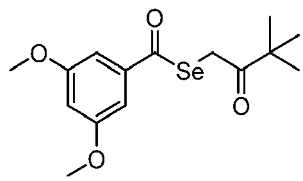

and 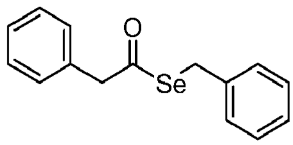 .

The present invention also relates to the salts and solvates of the compounds of formula **I**.

Some compounds of formula **I** can have chiral centers which can give rise to various stereoisomers. The present invention relates to each of these stereoisomers and also to mixtures thereof.

In another embodiment the invention relates to a compound of formula **I** for use in the treatment of microbial infections or colorectal cancers defined above, wherein $R_1$ represents -Ph$R_5$, -CO$R_5$, -S$R_5$, -(SO)$R_5$ or -($SO_2$)$R_5$.

In another embodiment the invention relates to a compound of formula **I** for use in the treatment of microbial infections or colorectal cancers as defined above, wherein $R_2$ represents -H.

In another embodiment the invention relates to a compound of formula **I** for use in the treatment of a microbial infection or colorectal cancers defined above, wherein each $R_4$ independently represents -$(CH_2)_t$H or-CN.

In another embodiment the invention relates to a compound of formula **I** for use in the treatment of microbial infections or colorectal cancers defined above, wherein t represents 1.

In another embodiment the invention relates to a compound of formula **I** for use in the treatment of microbial infections or colorectal cancers defined above, wherein n represents 0.

In another embodiment the invention relates to a compound of formula **I** for use in the treatment of microbial infections or colorectal cancers defined above, wherein:

$R_1$ represents -Ph$R_5$, -CO$R_5$, -S$R_5$, -(SO)$R_5$ or -($SO_2$)$R_5$; and
$R_2$ represents -H.

In another embodiment the invention relates to a compound of formula **I** for use in the treatment of microbial infections or colorectal cancers defined above, wherein:

$R_1$ represents $-PhR_5$, $-COR_5$, $-SR_5$, $-(SO)R_5$ or $-(SO_2)R_5$;
$R_2$ represents -H; and
each $R_4$ independently represents $-(CH_2)_tH$ or-CN.

In another embodiment the invention relates to a compound of formula **I** for use in the treatment of microbial infections or colorectal cancers defined above, wherein:

$R_1$ represents $-PhR_5$, $-COR_5$, $-SR_5$, $-(SO)R_5$ or $-(SO_2)R_5$; and
each $R_4$ independently represents $-(CH_2)_tH$ or-CN.

In another embodiment the invention relates to a compound of formula **I** for use in the treatment of microbial infections or colorectal cancers defined above, wherein:

$R_2$ represents -H; and
each $R_4$ independently represents $-(CH_2)_tH$ or-CN.

In another embodiment the invention relates to a compound of formula **I** for use in the treatment of microbial infections or colorectal cancers defined above, wherein:

t represents 1; and
n represents 0.

In another embodiment the invention relates to a compound of formula **I** for use in the treatment of microbial infections or colorectal cancers defined above, wherein:

$R_1$ represents $-PhR_5$, $-COR_5$, $-SR_5$, $-(SO)R_5$ or $-(SO_2)R_5$;
$R_2$ represents -H;
each $R_4$ independently represents $-(CH_2)_tH$ or-CN;
t represents 1; and
n represents 0.

In another embodiment the invention relates to a compound of formula **I** for use in the treatment of microbial infections or colorectal cancers as defined above, selected from:

*Se*-(cyanomethyl) 4-fluorobenzoselenoate (**1**);
*Se*-(cyanomethyl) 4-chlorobenzoselenoate (**3**);
*Se*-(cyanomethyl) benzoselenoate (**6**);
*Se*-(cyanomethyl) 4-methylbenzoselenoate (**7**);
*Se*-(cyanomethyl) 2-phenylethaneselenoate (**11**);
*Se*-(cyanomethyl) 2,4-dichlorobenzoselenoate (**13**);
*Se*-(cyanomethyl) 3,4-dichlorobenzoselenoate (**15**);
*Se*-(cyanomethyl) 2-acetoxybenzoselenoate (**17**);
*Se,Se*-bis-(cyanomethyl) benzene-1,4-bis-(carboselenoate) (**18**);
*Se,Se*-bis-(cyanomethyl) benzene-1,3-bis-(carboselenoate) (**19**);
*Se,Se,Se*-tris-(cyanomethyl) benzene-1,3,5-tris-(carboxyselenoate) (**20**);
*Se,Se*-bis-(2-oxopropyl) benzene-1,4-bis-(carboselenoate) (**22**);
*Se,Se*-bis-(2-oxopropyl) benzene-1,3-bis-(carboselenoate) (**23**); and
*Se,Se,Se*-tris-(2-oxopropyl) benzene-1,3,5-tris-(carboxyselenoate) (**24**).

In another embodiment the invention relates to a compound of formula **I** for use in the treatment of microbial infections or colorectal cancers as defined above, selected from:

*Se*-(cyanomethyl) 4-chlorobenzoselenoate (**3**);
*Se*-(cyanomethyl) 2-phenylethaneselenoate (**11**);
*Se*-(cyanomethyl) 2-acetoxybenzoselenoate (**17**);

*Se,Se,Se*-tris-(cyanomethyl) benzene-1,3,5-tris-(carboxyselenoate) (**20**);
*Se,Se*-bis-(2-oxopropyl) benzene-1,4-bis-(carboselenoate) (**22**); and
*Se,Se,Se*-tris-(2-oxopropyl) benzene-1,3,5-tris-(carboxyselenoate) (**24**).

In another embodiment the invention relates to a compound of formula **I** for use in the treatment of microbial infections caused by *Staphylococcus aureus,* methicillin resistant *Staphylococcus aureus, Candida spp* and/or *Escherichia coli* or colorectal cancers.

Another aspect of the present invention relates to a compound of formula **II**:

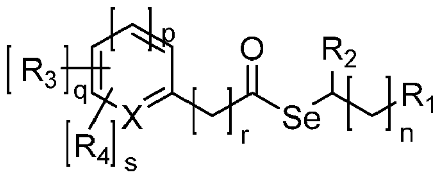

**II**

wherein:

$R_1$ represents -CN, $-PhR_5$, $-SR_5$, $-(SO)R_5$ or $-(SO_2)R_5$;
$R_2$ represents -H, $-CH_3$, $-CH_2CH_3$ or $-COCH_3$;
each $R_3$ independently represents $-[CH_2]_rCOSeCHR_2-[CH_2]_n-R_1$;
each $R_4$ independently represents -H, -F, -Cl, -Br, -I, $-(CH_2)_tH$, $-CH(CH_3)_2$,$-C(CH_3)_3$, -OH, $-OR_7$, $-OCOR_7$, $-SR_7$, $-(SO)R_7$, $-(SO_2)R_7$, -CN, $-CF_3$, $-COR_7$, -COOH, -Ph, $-NH_2$, $-NHR_6$, $-N(R_7)_2$, $-NO_2$;
each $R_5$ independently represents $-(CH_2)_tH$, $-CH(CH_3)_2$, $-C(CH_3)_3$, $-CF_3$, -Ph, $-PhR_4$;
each $R_6$ independently represents $-(CH_2)_tH$, $-CH(CH_3)_2$, $-C(CH_3)_3$, $-CF_3$, $-PhR_8$;
each $R_7$ independently represents $-(CH_2)_tH$, $-CH(CH_3)_2$, $-C(CH_3)_3$, -Ph;
$R_8$ represents -F, -Cl, -Br, -I, $-(CH_2)_tH$, $-CH(CH_3)_2$, $-C(CH_3)_3$, -OH, $-OR_7$, $-SR_7$,$-(SO)R_7$, $-(SO_2)R_7$, -CN, $-CF_3$, $-COR_7$, -COOH, -Ph, $-NH_2$, $-NH(R_6)_2$, $-N(R_7)_2$ or $-NO_2$;
t represents an integer that ranges between 1 and 6;
X represents CH, N or S;
p represents 0 or 1;
n represents an integer that ranges between 0 and 5;
q represents 0, 1 or 2;
r represents 0, 1 or 2; and
s represents an integer that ranges between 1 and 3,
with the proviso that the following compounds are excluded:

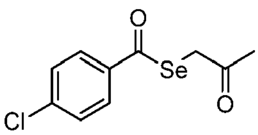
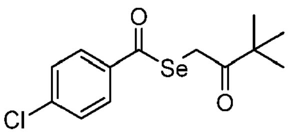
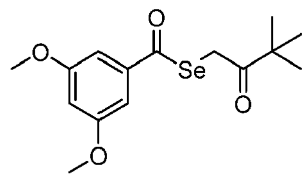
.

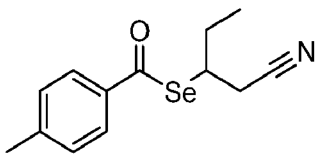
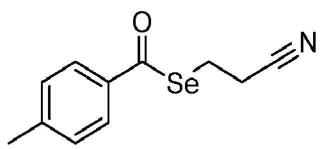
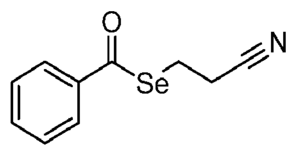

and 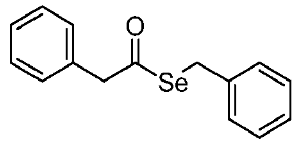
.

In another embodiment the invention relates to a compound of formula **II** as defined above, wherein $R_2$ represents -H.

In another embodiment the invention relates to a compound of formula **II** as defined above, wherein each $R_4$ independently represents $-(CH_2)_tH$ or-CN.

In another embodiment the invention relates to a compound of formula **II** as defined above, wherein t represents 1.

In another embodiment the invention relates to a compound of formula **II** as defined above, wherein n represents 0.

In another embodiment the invention relates to a compound of formula **II** selected from:

*Se*-(cyanomethyl) 4-fluorobenzoselenoate (**1**);
*Se*-(cyanomethyl) 4-chlorobenzoselenoate (**3**);
*Se*-(cyanomethyl) benzoselenoate (**6**);
*Se*-(cyanomethyl) 4-methylbenzoselenoate (**7**);
*Se*-(cyanomethyl) 2-phenylethaneselenoate (**11**);
*Se*-(cyanomethyl) 2,4-dichlorobenzoselenoate (**13**);
*Se*-(cyanomethyl) 3,4-dichlorobenzoselenoate (**15**);
*Se*-(cyanomethyl) 2-acetoxybenzoselenoate (**17**);
*Se,Se*-bis-(cyanomethyl) benzene-1,4-bis-(carboselenoate) (**18**);
*Se,Se*-bis-(cyanomethyl) benzene-1,3-bis-(carboselenoate) (**19**);
*Se,Se,Se*-tris-(cyanomethyl) benzene-1,3,5-tris-(carboxyselenoate) (**20**);
*Se,Se*-bis-(2-oxopropyl) benzene-1,4-bis-(carboselenoate) (**22**);
*Se,Se*-bis-(2-oxopropyl) benzene-1,3-bis-(carboselenoate) (**23**); and
*Se,Se,Se*-tris-(2-oxopropyl) benzene-1,3,5-tris-(carboxyselenoate) (**24**).

In another embodiment the invention relates to a compound of formula **II** selected from:

*Se*-(cyanomethyl) 4-chlorobenzoselenoate (**3**);
*Se*-(cyanomethyl) 2-phenylethaneselenoate (**11**);
*Se*-(cyanomethyl) 2-acetoxybenzoselenoate (**17**);
*Se,Se,Se*-tris-(cyanomethyl) benzene-1,3,5-tris-(carboxyselenoate) (**20**);
*Se,Se*-bis-(2-oxopropyl) benzene-1,4-bis-(carboselenoate) (**22**); and
*Se,Se,Se*-tris-(2-oxopropyl) benzene-1,3,5-tris-(carboxyselenoate) (**24**).

Another aspect of the present invention relates to a compound of formula **II** as defined above or a pharmaceutical salt thereof for use in therapy.

Another aspect of the present invention relates to a compound of formula **II** as defined above or a pharmaceutical salt thereof for use in the treatment of microbial infections, preferably for use in the treatment of microbial infections caused by *Staphylococcus aureus,* methicillin resistant *Staphylococcus aureus, Candida spp* and/or *Escherichia coli,* or colorectal cancer.

Another aspect of the present invention relates to a method of treating a microbial infections, preferably for use in the treatment of microbial infections caused by *Staphylococcus aureus,* methicillin resistant *Staphylococcus aureus, Candida spp* and/or *Escherichia coli,* or colorectal cancer in a subject in need thereof, especially a human being, which comprises administering to said subject an effective amount of a compound of formula **II** as defined above or a pharmaceutically acceptable salt thereof.

Another aspect of this invention relates to a pharmaceutical composition, which comprises a compound of formula **II** as defined above or a pharmaceutically acceptable salt thereof and one or more pharmaceutically acceptable excipients.

Another aspect of this invention relates to a pharmaceutical composition, which comprises a compound of formula **II** as defined above or a pharmaceutically acceptable salt thereof and one or more pharmaceutically acceptable excipients, for use in therapy.

Another aspect of this invention relates to a pharmaceutical composition, which comprises a compound of formula **II** as defined above or a pharmaceutically acceptable salt thereof and one or more pharmaceutically acceptable excipients, for use in the treatment of microbial infections, preferably for use in the treatment of microbial infections caused by *Staphylococcus aureus,* methicillin resistant *Staphylococcus aureus, Candida spp* and/or *Escherichia coli,* or colorectal cancer.

There is no limitation on the type of salt which can be used, provided that these are pharmaceutically acceptable when used for therapeutic purposes. The term pharmaceutically acceptable salt refers to those salts which are, according

to medical judgment, suitable for use in contact with the tissues of humans and other mammals without undue toxicity, irritation, allergic response and the like. Pharmaceutically acceptable salts are well known in the art.

The salts of a compound of formula **I** and **II** can be obtained during the final isolation and purification of the compounds of the invention or can be prepared by treating a compound of formula **I** and **II** with a sufficient amount of the desired acid or base to yield the salt in a conventional manner. The salts of the compounds of formula **I** and **II** can be converted into other salts of the compounds of formula **I** and **II** by ion exchange using ionic exchange resins.

The compounds of formula **I** and **II** and their salts may differ in some physical properties but they are equivalent for the purposes of the present invention. All salts of the compounds of formula **I** and **II** are included within the scope of the invention.

The compounds of the present invention may form complexes with solvents in which they are reacted or from which they are precipitated or crystallized. These complexes are known as solvates. As used herein, the term solvate refers to a complex of variable stoichiometry formed by a solute (a compound of formula **I** and **II** or a salt thereof) and a solvent. Examples of solvents include pharmaceutically acceptable solvents such as water, ethanol and the like. A complex with water is known as a hydrate. Solvates of compounds of the invention (or salts thereof), including hydrates, are included within the scope of the invention.

The compounds of formula **I** and **II** may exist in different physical forms, i.e. amorphous and crystalline forms. Moreover, the compounds of the invention may have the ability to crystallize in more than one form, a characteristic which is known as polymorphism. Polymorphs can be distinguished by various physical properties well known in the art such as X-ray diffraction pattern, melting point or solubility. All physical forms of the compounds of formula **I** and **II**, including all polymorphic forms ("polymorphs") thereof, are included within the scope of the invention.

Some of the compounds of the present invention may exist as several diastereoisomers and/or several optical isomers. Diastereoisomers can be separated by conventional techniques such as chromatography or fractional crystallization. Optical isomers can be resolved by conventional techniques of optical resolution to give optically pure isomers. This resolution can be carried out on any chiral synthetic intermediate or on products of formula **I** and **II**. Optically pure isomers can be obtained individually using enantiospecific synthesis. The present invention covers all individual isomers as well as mixtures thereof (for example racemic mixtures or mixtures of diastereomers), whether obtained by synthesis or by physically mixing them.

Unless defined otherwise, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples are provided by way of illustration and are not intended to limit of the present invention.

## EXAMPLES

### General synthetic procedure

A one-pot synthetic procedure with three reactions is followed to synthesize the compounds, as shown in Scheme 1. In a first step, an equivalent of selenium grey powder is suspended in 20 mL of water and over it are added slowly 2 equivalents of sodium borohydride. Mixture is left stirring at room temperature fifteen minutes after the end of the gas release. Afterwards, an equivalent of the corresponding acyl chloride is added and reaction is kept stirring during 90 min at 50-70°C. In some cases, the acyl chloride is poorly soluble in water and thus is necessary to add 8 mL of chloroform to perform the reaction in the interphase. Once completed the reaction, the mixture was filtered and an equivalent of the adequate alkyl halide was added to the filtrate, and mixture was left reacting till the end of the reaction (usually one hour at 50°C and one hour at room temperature). Compound was isolated and purified applying the appropriate techniques in each case (usually filtration and washings for solids; and for liquids, extraction and column chromatography). The proportions of the different reagents can vary for the derivatives that contain two or three selenium atoms. Acyl chlorides or alkyl halides, if not commercially available, can be synthesized using suitable chemical reactions, as for example the chlorination of the corresponding carboxylic acid with thionyl chloride, or the bromination of the appropriate starting material with *N*-bromosuccinimide, respectively.

In certain cases, the low yield obtained in the procedure described above makes necessary to carry out the reaction in absolute ethanol instead of in water, being the rest of the procedure similar but with slight modifications. For instance, the reaction of the selenium with sodium borohydride is cooled in ice bath when performed in absolute ethanol. And to isolate the compound after the reaction, then is necessary to evaporate the reaction mixture in rotary, and to treat the crude residue with dichloromethane. After removing solid impurities, compound is isolated by evaporation of the solvent in rotary, and then is purified using the most appropriate technique in each case.

*Scheme 1: General synthetic procedure of selenoesters*

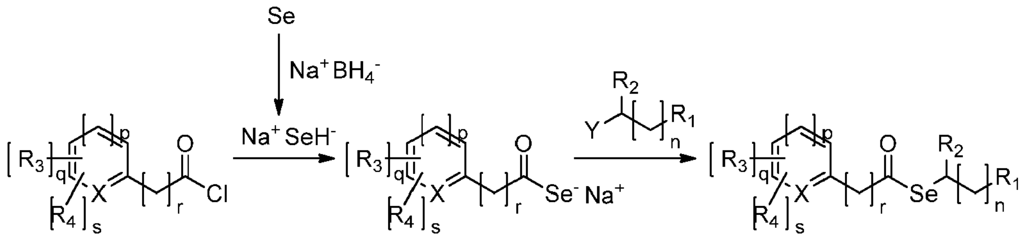


**I** or **II**

wherein $R_1$, $R_2$, $R_3$, $R_4$, X, p, n, q, r and s have the meaning previously described in relation with a compound of formula **I** or **II.**

***Se*-(cyanomethyl) 4-fluorobenzoselenoate (1)**

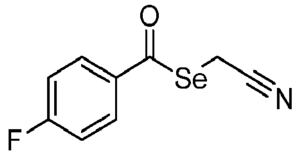

From sodium borohydride (0.395 g, 20.44 mmol), grey selenium (0.395 g, 5.00 mmol), 4-fluorobenzoyl chloride (0.6 mL, 5.08 mmol) and chloroacetonitrile (0.35 mL, 5.53 mmol). Chloroform addition was required. Final compound precipitated as a white solid that was isolated by filtration and washed with water, rendering 0.781 g (**65%**). **Mp**: 74-76 °C. **Elemental analysis, calculated/found (%)**: 44.65/44.63 C, 2.50/2.47 H, 5.79/5.77 N. **$^1$H-NMR (400 MHz, $CDCl_3$)** ppm: $\delta$ = 7.90 (dd, *J* = 5.1, 8.8 Hz, 2H, $H_{2Ph}$+$H_{6Ph}$); 7.19 (t, *J* = 8.5 Hz, 2H, $H_{3Ph}$+$H_{5Ph}$); 3.70 (s, 2H, $SeCH_2$).

***Se*-(cyanomethyl) 2-fluorobenzoselenoate (2)**

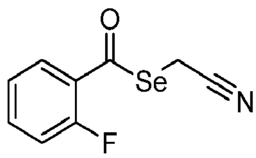

From sodium borohydride (0.197 g, 5.21 mmol), grey selenium (0.395 g, 2.50 mmol), 2-fluorobenzoyl chloride (0.3 mL, 2.51 mmol) and chloroacetonitrile (0.16 mL, 2.55 mmol). Chloroform addition was required. Final compound precipitated as a white solid that was isolated by filtration and washed with water, rendering 0.199 g (**33%**). **Mp**: 33-35 °C. **Elemental analysis, calculated/found (%)**: 44.65/44.54 C, 2.50/2.53 H, 5.79/5.81 N. **$^1$H-NMR (400 MHz, $CDCl_3$)** ppm: $\delta$ = 7.89 (td, *J* = 1.7, 7.9 Hz, 1H, $H_{6Ph}$); 7.63 (m, 1H, $H_{3Ph}$); 7.30 (td, *J* = 0.9, 7.9 Hz, 1H, $H_{4Ph}$); 7.22 (dd, *J* = 8.7, 11.14 Hz, 1H, $H_{5Ph}$); 3.68 (s, 2H, $SeCH_2$).

***Se*-(cyanomethyl) 4-chlorobenzoselenoate (3)**

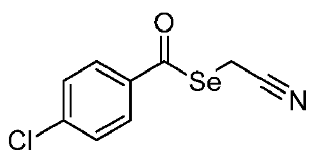

From sodium borohydride (0.475 g, 12.56 mmol), grey selenium (0.474 g, 6 mmol), p-chlorobenzoyl chloride (0.8 mL, 6.24 mmol) and chloroacetonitrile (0.6 mL, 9.48 mmol). Final compound precipitated as a white solid that was isolated by filtration and washed with water, rendering 0.510 g (**36%**). **Mp:** 68-69 °C. **Elemental analysis, calculated/found (%)**: 41.81/41.67 C, 2.34/ 2.34 H, 5.42/5.35 N. **$^1$H-NMR (400 MHz, $CDCl_3$)** ppm: $\delta$ = 7.80 (dt, *J* = 2.4, 8.6 Hz, 2H, $H_{2Ph}$+$H_{6Ph}$); 7.49 (dt, *J* = 2.3, 8.6 Hz, 2H, $H_{3Ph}$+$H_{5Ph}$); 3.70 (s, 2H, $SeCH_2$).

***Se*-(cyanoethyl) 4-chlorobenzoselenoate (4)**

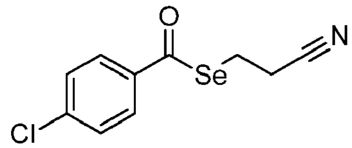

From sodium borohydride (0.475 g, 12.56 mmol), grey selenium (0.476 g, 6.02 mmol), p-chlorobenzoyl chloride (0.8 mL, 6.24 mmol) and 3-chloropropionitrile (0.5 mL, 6.39 mmol). Final compound precipitated and was filtered and purified by column chromatography (Hexane:Ethyl acetate 4:1) obtaining a beige solid, rendering 0.163 g (**12%**). **Mp**: 73-75 °C. **Elemental analysis calculated/found (%)**: 44.06/ 43.95 C, 2.96/2.01 H, 5.14/5.03 N. **$^1$H-NMR (400 MHz, $CDCl_3$)** ppm: $\delta$ = 7.82 (m, 2H, $H_{2Ph}$+$H_{6Ph}$); 7.45 (m, 2H, $H_{3Ph}$+$H_{5Ph}$); 3.27 (dt, *J* = 2.1, 7.1 Hz, 2H, $SeCH_2$); 2.87 (dt, *J* = 1.5, 7.1 Hz, 2H, $CH_2CN$).

***Se*-(cyanopropyl) 4-chlorobenzoselenoate (5)**

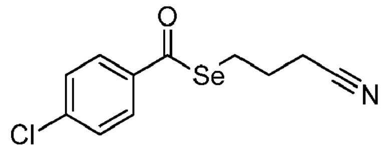

From sodium borohydride (0.358 g, 9.46 mmol), grey selenium (0.357 g, 4.52 mmol), p-chlorobenzoyl chloride (0.6 mL, 4.68 mmol) and 4-chlorobutironitrile (0.45 mL, 4.76 mmol). Extraction with dichloromethane (4x30 mL) was required. After solvent evaporation, the crude oil was purified by column chromatography using a eluent gradient (Dichloromethane:Hexane 3:1 $\rightarrow$ 1:0) obtaining a transparent oil, rendering 0.104 g (**8%**). **Elemental analysis calculated/found (%)**: 46.10/ 45.89 C, 3.52/3.40 H, 4.86/4.87 N. **$^1$H-NMR (400 MHz, $CDCl_3$)** ppm: $\delta$ = 7.83 (dt, *J* = 2.2, 9.0, 2H, $H_{2Ph}$+$H_{6Ph}$); 7.44 (dt, *J* = 2.2, 9.0, 2H, $H_{3Ph}$+$H_{5Ph}$); 3.19 (t, *J* = 7.0 Hz, 2H, $SeCH_2$); 2.49 (t, *J* = 7.2 Hz, 2H, $CH_2CN$), 2.13 (quin, *J* = 7.1 Hz, 2H, $CH_2\underline{CH_2}CH_2$).

***Se*-(cyanomethyl) benzoselenoate (6)**

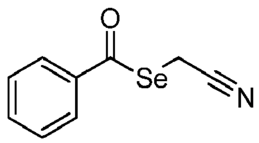

From sodium borohydride (0.474 g, 12.52 mmol), grey selenium (0.474 g, 6.00 mmol), benzoyl chloride (0.7 mL, 6 mmol) and chloroacetonitrile (0.4 mL, 6.32 mmol). Final compound was precipitated and purified by column chromatography (Dichloromethane:Hexane 3:1) obtaining a yellow solid, rendering 0.195 g (**14%**). **Mp:** 32-34 °C. **Elemental analysis calculated/found (%)**: 48.23/47.93 C, 3.15/3.15 H, 6.25/6.35 N. **$^1$H-NMR (400 MHz, $CDCl_3$)** ppm: $\delta$ = 7.86 (d, *J* = 8.3 Hz, 2H, $H_{2Ph}$+$H_{6Ph}$); 7.7 (t, *J* = 7.4 Hz, 1H, $H_{4Ph}$); 7.5 (t, *J* = 7.7 Hz, 2H, $H_{3Ph}$+$H_{5Ph}$); $\delta$ = 3.70 (s, 2H, $SeCH_2$).

***Se*-(cyanomethyl) 4-methylbenzoselenoate (7)**

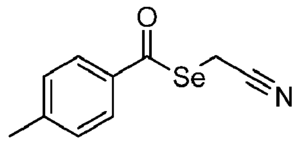

From sodium borohydride (0.476 g, 12.58 mmol), grey selenium (0.475 g, 6.02 mmol), p-toluoyl chloride (0.8 mL, 6.05 mmol) and chloroacetonitrile (0.6 mL, 6.60 mmol). Chloroform addition was required. Final compound precipitated as a beige solid that was isolated by filtration and washed with water, rendering 0.202 g (**14%**). **Mp:** 51-53 °C. **Elemental analysis, calculated/found (%)**: 50.44/50.17 C, 3.81/3.75 H, 5.88/5.79% N. **$^1$H-NMR (400 MHz, $CDCl_3$)** ppm: $\delta$ = 7.76 (d, *J* = 8.2 Hz, 2H, $H_{2Ph}$+$H_{6Ph}$); 7.30 (d, *J* = 8.0 Hz, 2H, $H_{3Ph}$+$H_{5Ph}$); 3.68 (s, 2H, $SeCH_2$); 2.43 (s, 3H, Ph-$CH_3$).

***Se*-(cyanoethyl) 4-methylbenzoselenoate (8)**

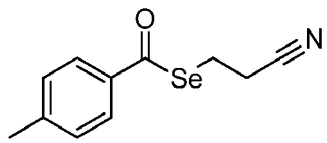

From sodium borohydride (0.355 g, 9.38 mmol), grey selenium (0.355 g, 4.50 mmol), p-toluoyl chloride (0.6 mL, 4.54 mmol) and 3-chloropropionitrile (0.5 mL, 6.39 mmol). Chloroform was required. The mixture was heated at 70°C for 2 hours. Final compound precipitated and the impure solid was purified by column chromatography using a eluent gradient (Dichloromethane:Hexane 3:1 $\rightarrow$ 1:0) obtaining a white solid, rendering 0.084 g (**7%**). **Mp**: 41-43 °C. **Elemental analysis calculated/found (%)**: 52.39/52.18 C, 4.40/4.83 H, 5.55/5.55 N. **$^1$H-NMR (400 MHz, $CDCl_3$)** ppm: $\delta$ = 7.71 (d, *J* = 8.3 Hz, 2H, $H_{2Ph}$+$H_{6Ph}$); 7.20 (d, *J* = 8.5 Hz, 2H, $H_{3Ph}$+$H_{5Ph}$); 3.25 (t, *J* = 7.1 Hz, 2H, $SeCH_2$); 2.86 (t, *J* = 7.1 Hz, 2H, $CH_2CN$); 2.43 (s, 3H, Ph-$CH_3$).

***Se*-(cyanopropyl) 4-methylbenzoselenoate (9)**

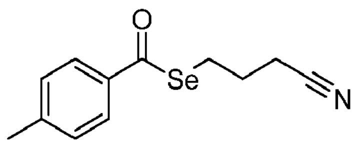

From sodium borohydride (0.356 g, 9.41 mmol), grey selenium (0.355 g, 4.50 mmol), p-toluoyl chloride (0.6 mL, 4.54 mmol) and 4-chlorobutironitrile (0.45 mL, 4.76 mmol). Chloroform was required. The product was extracted with dichloromethane (3x40 mL), dried over $Na_2SO_4$ and concentrated to dryness. This compound purified was by column chromatography (Dichloromethane) obtaining a yellow oil **9** (0.064 g, **5%**). **Elemental analysis calculated/found (%)**: 54.14/ 53.89 C, 4.92/5.05 H, 5.26/5.29 N. **$^1$H-NMR (400 MHz, $CDCl_3$)** ppm: $\delta$ = 7.78 (d, *J* = 8.2, 2H, $H_{2Ph}$+$H_{6Ph}$); 7.24 (d, *J* = 8.2, 2H, $H_{3Ph}$+$H_{5Ph}$); 3.15 (t, *J* = 7.0 Hz, 2H, $SeCH_2$); 2.48 (t, *J* = 7.2 Hz, 2H, $CH_2CN$), 2.11 (quin, *J* = 7.1 Hz, 2H, $CH_2\underline{CH_2}CH_2$).

***Se*-(cyanomethyl) 4-*tert*-butylbenzoselenoate (10)**

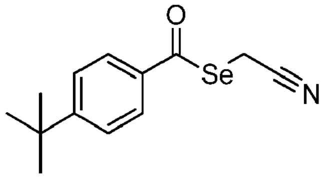

Synthesis in absolute ethanol. From sodium borohydride (0.400 g, 10.57 mmol), grey selenium (0.399 g, 5.05 mmol), p-*tert*-butylbenzoyl chloride (1.0 mL, 5.12 mmol) and chloroacetonitrile (0.35 mL, 5.54 mmol). The crude residue obtained after the reaction and after initial purification was purified by column chromatography (Dichloromethane:Hexane 2:1) obtaining **10** (0.333 g, **33%**) as a yellow oil. **Elemental analysis, calculated/found (%)**: 55.72/55.51 C, 5.40/5.31 H, 5.00/5.07 N. **$^1$H-NMR (400 MHz, $CDCl_3$)** ppm: $\delta$ = 7.80 (m, 2H, $H_{2Ph}$+$H_{6Ph}$); 7.51 (m, 2H, $H_{3Ph}$+$H_{5Ph}$); 3.68 (s, 2H, $SeCH_2$); 1.35 (s, 9H, $C(CH_3)_3$).

***Se*-(cyanomethyl) 2-phenylethaneselenoate (11)**

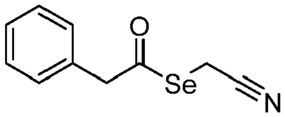

From sodium borohydride (0.476 g, 12.58 mmol), grey selenium (0.476 g, 6.03 mmol), phenylacetyl chloride (0.8 mL, 6.05 mmol) and chloroacetonitrile (0.4 mL, 6.32 mmol). Chloroform addition was required. The product was extracted with dichloromethane (4x30 mL), dried over $Na_2SO_4$ and concentrated to dryness. Crude oil was then purified by column chromatography (Dichloromethane) obtaining a transparent oil (**11**, 0.051 g, **4%**). **Elemental analysis calculated/found (%)**: 50.44/50.05 C, 3.81/3.69 H, 5.88/5.81 N. **$^1$H-NMR (400 MHz, $CDCl_3$)** ppm: $\delta$ = 7.39 (m, 3H, $H_{3Ph}$+$H_{4Ph}$+$H_{5Ph}$); 7.30 (m, 2H, $H_{2Ph}$+$H_{6Ph}$); 3.90 (s, 2H, $PhCH_2$); 3.43 (s, 2H, $SeCH_2$).

***Se*-(cyanomethyl) 2,4-difluorobenzoselenoate (12)**

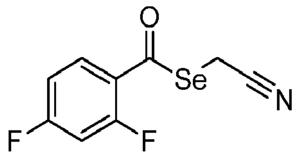

From sodium borohydride (0.197 g, 5.21 mmol), grey selenium (0.197 g, 2.50 mmol), 2,4-difluorobenzoyl chloride (0.3 mL, 2.55 mmol) and chloroacetonitrile (0.16 mL, 2.53 mmol). Chloroform addition was required. Final compound precipitated as a white solid and was isolated by filtration to obtain **12** (0.255 g, **39%**). **Mp:** 58-60 °C. **Elemental analysis, calculated/found (%)**: 41.56/41.32 C, 1.94/1.90 H, 5.39/5.41 N. **$^1$H NMR (400 MHz, $CDCl_3$)** ppm: $\delta$ = 7.94 (td, *J* = 6.3, 8.5 Hz, 1H, $H_{6Ph}$); 7.03 (m, 1H, $H_{3Ph}$); 6.96 (ddd, *J* = 2.4, 8.4, 10.9 Hz, 1H, $H_{5Ph}$); 3.65 (s, 2H, $SeCH_2$).

***Se*-(cyanomethyl) 2,4-dichlorobenzoselenoate (13)**

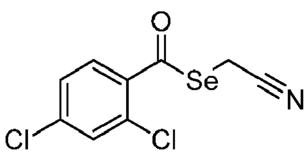

From sodium borohydride (0.360 g, 9.52 mmol), grey selenium (0.357 g, 4.52 mmol), 2,4-dichlorobenzoyl chloride (0.65 mL, 4.24 mmol) and chloroacetonitrile (0.3 mL, 4.74 mmol). Final compound precipitated and was isolated

by filtration as a white solid **13** (0.481 g, **36%**). **Mp:** 55-57 °C. **Elemental analysis, calculated/found (%)**: 36.89/36.76 C, 1.72/1.88 H, 4.78/4.77 N. **$^1$H NMR (400 MHz, $CDCl_3$)** ppm: $\delta$ = 7.65 (d, *J* = 8.5 Hz, 1H, $H_{6Ph}$); 7.45 (t, *J* = 2.1 Hz, 1H, $H_{3Ph}$); 7.32 (dt, *J* = 1.5, 8.5 Hz, 1H, $H_{5Ph}$); 3.63 (s, 2H, $SeCH_2$).

***Se*-(cyanoethyl) 2,4-dichlorobenzoselenoate (14)**

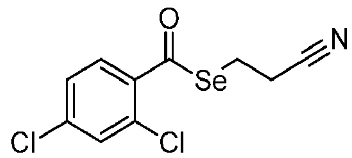

From sodium borohydride (0.358 g, 9.46 mmol), grey selenium (0.357 g, 4.52 mmol), 2,4-dichlorobenzoyl chloride (0.65 mL, 4.64 mmol) and 3-chloropropionitrile (0.35 mL, 4.73 mmol). Chloroform addition was required. The product precipitated and was purified by column chromatography (Dichloromethane) obtaining a transparent oil **14** (0.057 g, **4%**). **Elemental analysis calculated/found (%)**: 39.12/38.95 C, 2.30/2.20 H, 4.56/4.53 N. **$^1$H-NMR (400 MHz, $CDCl_3$)** ppm: $\delta$ = 7.68 (d, *J* = 8.4 Hz, 1H, $H_{6Ph}$); 7.49 (d, *J* = 1.9 Hz, 1H, $H_{3Ph}$); 7.36 (dd, *J* = 2.0, 8.4 Hz, 1H, $H_{5Ph}$); 3.27 (t, *J* = 7.0 Hz, 2H, $SeCH_2$); 2.89 (t, *J* = 7.0 Hz, 2H, $CH_2CN$).

***Se*-(cyanomethyl) 3,4-dichlorobenzoselenoate (15)**

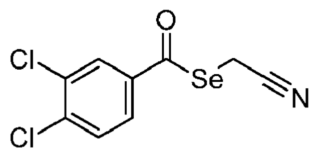

From sodium borohydride (0.357 g, 9.43 mmol), grey selenium (0.356 g, 4.51 mmol), 3,4-dichlorobenzoyl chloride (0.952 g, 4.55 mmol) and chloroacetonitrile (0.3 mL, 4.74 mmol). Chloroform addition was required. Final compound precipitated as a white solid which was isolated by filtration as **15** (0.328 g, **25%**). **Mp:** 76-77 °C. **Elemental analysis, calculated/found (%)**: 36.89/36.76 C, 1.72/1.88 H, 4.78/4.82 N. **$^1$H-NMR (400 MHz, $CDCl_3$)** ppm: $\delta$ = 7.94 (d, *J* = 1.9 Hz, 1H, $H_{2Ph}$); 7.69 (dd, *J* = 2.1, 8.4 Hz, 1H, $H_{5Ph}$); 7.60 (d, *J* = 8.4 Hz, 1H, $H_{6Ph}$); 3.72 (s, 2H, $SeCH_2$).

**Se-(cyanoethyl) 3,4-dichlorobenzoselenoate (16)**

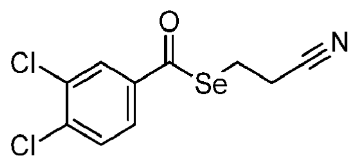

Synthesis in absolute ethanol from sodium borohydride (0.360 g, 9.52 mmol), grey selenium (0.358 g, 4.53 mmol), 3,4-dichlorobenzoyl chloride (0.946 g, 4.52 mmol) and 3-chloropropionitrile (0.36 mL, 4.60 mmol). The crude residue obtained after the reaction and after initial purification was purified by column chromatography (Dichloromethane:Hexane 4:1), obtaining **16** (0.333 g, **33%**) as a grey solid. **Mp**: 35-38°C. **Elemental analysis, calculated/found (%)**: 39.12/38.92 C, 2.30/2.331 H, 4.56/4.394% N. **$^1$H-NMR (400 MHz, $CDCl_3$)** ppm: $\delta$ = 7.95 (d, *J* = 2.1 Hz, 1H, $H_{2Ph}$); 7.71 (dd, *J* = 2.1, 8.4 Hz, 1H, $H_{5Ph}$); 7.57 (d, *J* = 8.4 Hz, 1H, $H_{6Ph}$); 3.29 (t, *J* = 7.0 Hz, 2H, $SeCH_2$); 3.29 (t, *J* = 7.0 Hz, 2H, $CH_2CN$).

***Se*-(cyanomethyl) 2-acetoxybenzoselenoate (17)**:

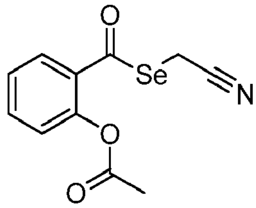

From sodium borohydride (0.399 g, 10.55 mmol), grey selenium (0.396 g, 5.01 mmol), o-acetylsalicyloyl chloride (0.997 g, 5.02 mmol) and chloroacetonitrile (0.35 mL, 5.54 mmol). A viscous oil was formed after the reaction, which was extracted with dichlorometane. The evaporation of the organic fraction rendered a solid which was purified by column chromatography (Dichloromethane), obtaining **17** (0.094 g, **13%**) as a white crystalline solid. **Mp**: 49-51 °C. **Elemental analysis, calculated/found (%)**: 46.83/46.56 C, 3.22/3.17 H, 4.96/5.04 N. **$^1$H-NMR (400 MHz, $CDCl_3$)** (ppm): $\delta$ = 7.89 (dd, *J* = 1.6, 7.9 Hz, 1H, $H_{6Ph}$); 7.65 (td, *J* = 1.6, 8.1 Hz, 1H, $H_{4Ph}$); 7.39 (td, *J* = 1.1, 7.7 Hz, 1H, $H_{3Ph}$); 7.21 (dd, *J* = 1.1, 8.1 Hz, 1H, $H_{5Ph}$); 3.64 (s, 2H, $SeCH_2$); 2.41 (s, 3H, $OCOCH_3$).

***Se,Se*-bis-(cyanomethyl) benzene-1,4-bis-(carboselenoate) (18)**

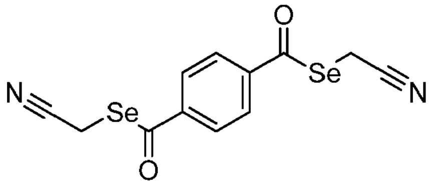

From terephthaloyl chloride (0.64 g, 3.2 mmol), grey selenium (0.51 g, 6.5 mmol), sodium borohydride (0.51g, 13.5 mmol) and chloroacetonitrile (0.47 g, 6.5 mmol). The precipitate was filtered, washed with water and dried to obtain a yellow solid (**18**, 0.346 g, **30%**). **Mp:** 166-167 °C. **Elemental analysis, calculated/found (%)**: C: 38.94/38.62; H: 2.18/2.32; N: 7.57/7.54. **$^1$H-NMR (300 MHz, $CDCl_3$)** ppm: $\delta$ = 8.00 (s, 4H, $H_{2Ph}$+$H_{3Ph}$+$H_{4Ph}$+$H_{6Ph}$), 3.75 (s, 4H, $CH_{2(Se)}$).

***Se,Se*-bis-(cyanomethyl)benzene-1,3-bis-(carboselenoate) (19)**

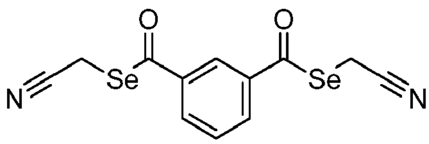

From isophthaloyl chloride (0.65 g, 3.2mmol), grey selenium (0.50 g, 6.33 mmol), sodium borohydride (0.50 g, 13.2 mmol) and chloroacetonitrile (0.68 g, 9.0 mmol). The precipitate was filtered, washed with water and dried to obtain a light orange solid (**19**, 0.795 g, **68%**). **Mp:** 95-97 °C. **Elemental analysis, calculated/found (%)**: C: 38.94/38.64; H: 2.18/2.32; N: 7.57/7.74. **$^1$H-NMR (300 MHz, $CDCl_3$)** ppm: $\delta$ = 8.32 (t, 1H, *J* = 1.8 Hz, $H_{2Ph}$), 8.15 (dd, 2H, *J* = 7.8 Hz, *J* = 1.8 Hz, $H_{4Ph}$+$H_{6Ph}$), 7.71 (t, 1H, *J* = 7.8 Hz, $H_{5Ph}$), 3.76 (s, 4H, $CH_{2(Se)}$).

***Se,Se,Se*-tris-(cyanomethyl) benzene-1,3,5-tris-(carboxyselenoate) (20)**

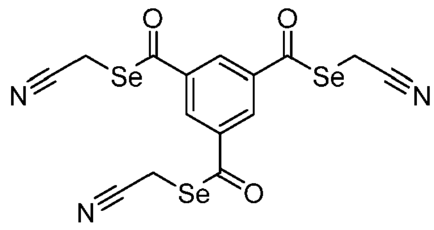

From 1,3,5-benzenetricarbonyl chloride (0.80 g, 3.0 mmol), grey selenium (0.71 g, 9.0 mmol), sodium borohydride (0.71g, 18.9 mmol) and chloroacetonitrile (1.06 g, 14 mmol). The precipitate was filtered, washed with water and dried to obtain a yellow-orange solid (**20**, 0.945 g, **61%**). **Mp:** 116-119 °C. **Elemental analysis**, **calculated/found** (%): C: 34.91/34.54; H: 1.76/1.96; N: 8.14/8.31. **$^1$H-NMR (300 MHz, $CDCl_3$)** ppm: $\delta$ = 8.53 (s, 3H, $H_{1Ph}$+$H_3p_h$+$H_{5Ph}$), 3.81 (s, 6H, $CH_{2(Se)}$).

***Se*-(2-oxopropyl) 4-fluorobenzoselenoate (21)**

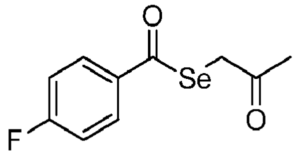

From sodium borohydride (0.396 g, 10.47 mmol), grey selenium (0.399 g, 5.05 mmol), p-fluorobenzoyl chloride (0.6 mL, 5.08 mmol) and chloroacetone (0.41 mL, 4.99 mmol). Final compound precipitated as yellow oil that was isolated by decantation, rendering 0.190 g of **21 (15%)**. **Elemental analysis, calculated/found (%)**: 46.35/46.38 C, 3.50/3.66 H. **$^1$H-RMN (300 MHz, $CDCl_3$, $\delta$):** 7.93 (dd, *J*= 8.9, 5.2 Hz, 2H, $H_{2Ph}$+$H_{6Ph}$), 7.16 (t, *J* = 8.6 Hz, 2H, $H_{3Ph}$+$H_{5Ph}$), 3.91 (s, 2H, $SeCH_2$), 2.34 (s, 3H, $CH_3$).

***Se,Se*-bis-(2-oxopropyl) benzene-1,4-bis-(carboselenoate) (22)**

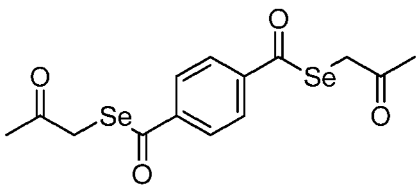

From terephthaloyl chloride (1.30 g, 6.4 mmol), grey selenium (1.04 g, 12.7 mmol), sodium borohydride (1.03 g, 26.4 mmol) and chloroacetone (1.17g, 12.7 mmol). The precipitate was filtered, washed with water and dried to obtain a yellow solid (**22**, 1.793 g, **70%**). **Mp:** 101-102 °C. **Elemental analysis, calculated/found (%)**: C: 41.60/41.47; H: 3.49/3.51. **$^1$H-NMR (300 MHz, $CDCl_3$)** ppm: $\delta$ = 8.00 (s, 4H, $H_{2Ph}$+$H_{3Ph}$+$H_{5Ph}$+$H_{6Ph}$), 3.97 (s, 4H, $CH_{2(Se)}$), 2.37 (s, 6H, $CH_{3(CO)}$).

**Se,Se-bis-(2-oxopropyl) benzene-1,3-bis-(carboselenoate) (23)**

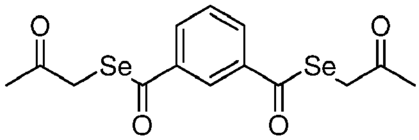

From isophthaloyl chloride (0.50 g, 2.5 mmol), grey selenium (0.41g, 5.2 mmol), sodium borohydride (0.41 g, 10.8 mmol) and chloroacetone (0.46 g, 5.0 mmol). The precipitate was filtered, washed with water and dried to obtain a yellow solid (**23**, 0.549 g, **54%**). **Mp:** 50-52 °C. **Elemental analysis, calculated/found (%)**: C: 41.60/41.44; H: 3.49/3.53; **$^1$H-NMR (300 MHz, $CDCl_3$)** ppm: $\delta$ = 8.40 (d, 1H, *J* = 1.3 Hz, $H_{2Ph}$), 8.14 (c, 2H, *J* = 7.8 Hz, *J* = 1.7 Hz, $H_{4Ph}$+$H_{6Ph}$), 7.63 (t, 1H, *J* = 7.8 Hz, $H_{5Ph}$), 3.97 (s, 4H, $CH_{2(Se)}$), 2.36 (s, 6H, $CH_{3(CO)}$).

***Se,Se,Se*-tris-(2-oxopropyl) benzene-1,3,5-tris-(carboxyselenoate) (24)**

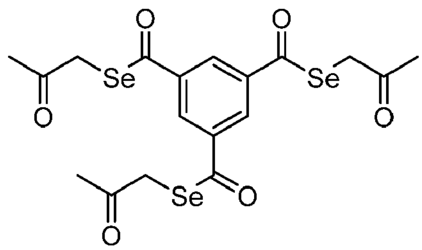

From 1,3,5-benzenetricarbonyl chloride (0.54 g, 2.0 mmol), grey selenium (0.49 g, 6.1 mmol), sodium borohydride (0.49 g, 12.8 mmol) and chloroacetone (0.83 g, 9.0 mmol). The precipitate was filtered, washed with water and dried to obtain a red solid. (**24**, 0.707 g, **62%**). **Mp:** 87-89 °C. **Elemental analysis, calculated/found** (%): C: 38.12/37.84; H: 3.20/3.28.**$^1$H-NMR (300 MHz, $CDCl_3$)** ppm: $\delta$ = 8.57 (s, 3H, $H_{1Ph}$+$H_{3Ph}$+$H_{5Ph}$), 4.02 (s, 6H, $CH_{2(Se)}$), 2.39 (s, 9H, $CH_{3(CO)}$).

***Se*-(3,3-dimethyl-2-oxobutyl) 4-fluorobenzoselenoate (25)**

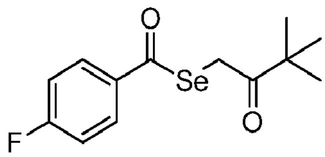

From sodium borohydride (0.198 g, 5.23 mmol), grey selenium (0.196 g, 2.48 mmol), p-fluorobenzoyl chloride (0.30 mL, 2.54 mmol) and 1-chloropinacolone (0.33 mL, 2.51 mmol). Final compound precipitated as a light yellow solid that was isolated by filtration and washed with water, rendering 0.356 g of **25** (**47%**). **Mp**: 58-60 °C. **Elemental analysis, calculated/found** (%): 51.84/51.79 C, 5.02/4.95 H. **$^1$H-RMN (300 MHz, $CDCl_3$, $\delta$)**: 7.93 (dd, *J*= 8.9, 5.2 Hz, 2H, $H_{2Ph}$+$H_{6Ph}$), 7.14 (t, *J*= 8.6 Hz, 2H, $H_{3Ph}$+$H_{5Ph}$), 4.16 (s, 2H, $SeCH_2$), 1.27 (s, 9H, $3CH_3$).

***Se,Se*-bis-(3,3-dimethyl-2-oxobutyl)benzene-1,4-bis-(carboselenoate) (26)**

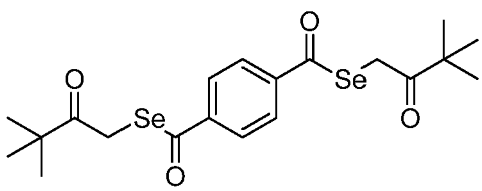

From terephthaloyl chloride (0.64 g, 3.2 mmol), grey selenium (0.51 g, 6.4 mmol), sodium borohydride (0.51 g, 13.5 mmol) and chloropinacolone (0.85 g, 6.3 mmol). The precipitate was filtered, washed with water and dried to obtain a yellow solid. (**26**, 1.143 g, **74%**). **Mp:** 142-144 °C. **Elemental analysis, calculated/found (%)**: C: 49.19/48.86; H: 5.37/5.21. **$^1$H-NMR (300 MHz, $CDCl_3$)** ppm: $\delta$ = 7.97 (s, 4H, $H_{2Ph}$+$H_{3Ph}$+$H_{5Ph}$+$H_{6Ph}$), 4.21 (s, 4H, $CH_{2(Se)}$), 2.28 (s, 18H, $CH_3$).

**Evaluation of the cytotoxic activity and assessment of the selectivity. Methodology.**

Cytotoxicity assays in human colonic adenocarcinoma cell lines Colo 205 doxorubicin-sensitive (ATCC-CCL-222) and Colo 320/MDR-LRP multidrug resistant overexpressing ABCB1 (MDR1)-LRP (ATCC-CCL-220.1) were performed following the procedure described by Gajdács et al, 2017, Bioorg Med Chem Lett. 27(4): 797-802. The effects of increasing concentrations of selenium compounds on cell growth were tested in 96-well flat-bottomed microtiter plates. The selectivity of the action towards cancer cells was assessed through the evaluation of cytotoxic activity in a non-tumoral cell line, the MRC-5 human embryonal lung fibroblast cell lines (ATCC CCL-171), as described in Gajdács et al, 2017, Bioorg Med Chem Lett. 27(4): 797-802.

All cell lines were purchased from LGC Promochem, Teddington, UK. The colonic cells were cultured in RPMI 1640 medium supplemented with 10% heat-inactivated fetal bovine serum, 2 mM L-glutamine, 1 mM Na-pyruvate and 100 mM Hepes. The cell lines were incubated at 37°C, in a 5% $CO_2$, 95% air atmosphere. The semi-adherent human colon cancer cells were detached with Trypsin-Versene (EDTA) solution for 5 min at 37°C. Alternatively, MRC-5 cells was cultured in Eagle's Minimal Essential Medium (EMEM, containing 4.5 g/L glucose) supplemented with a non-essential amino acid mixture, a selection of vitamins and 10% heat-inactivated fetal bovine serum. The cell lines were incubated at 37°C, in a 5% $CO_2$, 95% air atmosphere.

The compounds were diluted in a volume of 100 μL of medium. The two-fold serial dilutions of selenocompounds were prepared in 100 μL of RPMI 1640, horizontally. The semi-adherent colonic adenocarcinoma cells were treated with Trypsin-Versene (EDTA) solution. They were adjusted to a density of $2\times10^4$ cells in 100 μL of RPMI 1640 medium, and were added to each well, with the exception of the medium control wells. The final volume of the wells containing selenocompounds and cells was 200 μL.

The culture plates were incubated at 37°C for 24 h; at the end of the incubation period, 20 μL of MTT (thiazolyl blue tetrazolium bromide, Sigma) solution (from a stock solution of 5 mg/mL) were added to each well. After incubation at 37°C for 4 h, 100 μL of sodium dodecyl sulfate (SDS) (Sigma) solution (10% in 0.01 M HCl) were added to each well and the plates were further incubated at 37°C overnight. Cell growth was determined by measuring the optical density (OD) at 540/630 nm with Multiscan EX ELISA reader (Thermo Labsystems, Cheshire, WA, USA). Inhibition of the cell growth was determined according to the formula below:

$$IC_{50} = 100 - \left[\frac{OD\,sample - OD\,medium\,control}{OD\,cell\,control - OD\,medium\,control}\right] \times 100$$

Results are expressed in terms of $IC_{50}$, defined as the inhibitory dose that reduces the growth of the cells exposed to the tested compounds by 50%. Regarding the selectivity, the selectivity indexes (SI) were calculate, being SI the quotient of the $IC_{50}$ in non-tumoral cells (MRC-5) and the respective cancer cell (Colo-205 or Colo-320). Compound is considered moderately selective when SI < 6 and ≥ 3; and strongly selective when SI ≥ 6.

**Cytotoxicity and selectivity of the selenocompounds.**

$IC_{50}$ values have been calculated as mentioned above in the methodology of evaluation of the cytotoxic activity and assessment of the selectivity. To express the cytotoxicity results in Table 2, four intervals have been defined in function of the $IC_{50}$ values:

- Very active compounds: $IC_{50} \leq 1$ μM. Signalled as ***.
- Active compounds: 1 μM < $IC_{50} \leq 10$ μM. Signalled as **.
- Slightly active compounds: 10 μM < $IC_{50} \leq 50$ μM. Signalled as *.
- Not cytotoxic compounds: $IC_{50} \geq 50$ μM. Signalled as NC.

For the selectivity, the following intervals have been defined depending of the SI values:

- Strongly selective compounds: SI ≥ 6. Signalled as ***.
- Moderately selective compounds: 3 ≤ SI < 6. Signalled as **.
- Slightly selective compounds: 1 ≤ SI < 3. Signalled as *.
- Not selective compounds: $IC_{50} \geq 50$ μM. Signalled as NS.

*Table 2: Cytotoxicity and selectivity of the selenoesters.*

| Compound | Colo 205 Cytotoxicity | Colo 320 Cytotoxicity | MRC-5 Cytotoxicity | SI (MRC-5/Colo205) | SI (MRC-5/Colo320) |
|---|---|---|---|---|---|
| 1 | ** | ** | ND | ND | ND |
| 3 | *** | ** | ND | ND | ND |
| 4 | ** | ** | ND | ND | ND |
| 5 | ** | ** | ND | ND | ND |
| 6 | ** | ** | ND | ND | ND |
| 7 | ** | ** | ND | ND | ND |
| 8 | ** | ** | ND | ND | ND |
| 9 | ** | ** | ND | ND | ND |
| 10 | ** | ** | ND | ND | ND |
| 11 | *** | *** | ND | ND | ND |
| 13 | ** | ** | ND | ND | ND |
| 14 | ** | ** | ND | ND | ND |
| 15 | ** | ** | ND | ND | ND |
| 17 | *** | ** | ND | ND | ND |
| 18 | ** | ** | NC | *** | *** |
| 19 | ** | ** | NC | *** | *** |
| 20 | *** | ** | NC | *** | *** |
| 22 | *** | *** | *** | * | * |
| 23 | *** | *** | ** | * | * |
| 24 | *** | *** | *** | * | * |

ND: Not determined.

**Evaluation of the inhibition of the multidrug resistance efflux pumps. Methodology.**

This method, described in Gajdács et al, 2017, Bioorg Med Chem Lett. 27(4): 797-802, is a fluorescence-based detection system which uses verapamil as reference inhibitor of the ABCB1 efflux pump. The colonic adenocarcinoma cells (the doxorubicin-sensitive Colo 205 and the multidrug resistant Colo 320) were adjusted to a density of $2\times10^6$/mL, re-suspended in serum-free RPMI 1640 medium and distributed in 0.5 mL aliquots into Eppendorf centrifuge tubes. Selenocompounds (1 and 10 $\mu$L from a stock solution of 1 mM, respectively) were added at different concentrations (2 $\mu$M and 20 $\mu$M final concentrations, respectively), and the samples were incubated for 10 min at room temperature. Next, 10 $\mu$L (5.2 $\mu$M final concentration) of rhodamine 123 was added to the samples and the cells were incubated for 20 minutes at 37°C, washed twice with phosphate buffered saline (PBS) and re-suspended in 1 mL PBS for analysis. The fluorescence intensity of the cell population was measured with a Partec CyFlow flow cytometer (Partec, Munster, Germany). Verapamil was used as a positive control at 20 $\mu$M final concentration in the rhodamine 123 exclusion experiments. The mean fluorescence intensity (%) was calculated for the treated multidrug resistance (MRD) Colo 320 and sensitive Colo 205 cell lines as compared to the untreated cells. The fluorescence activity ratio (FAR) was calculated based on the following equation which relates the measured fluorescence values:

$$FAR = \frac{MDR_{treated} / MDR_{control}}{parental_{treated} / parental_{control}} \text{ ; Quotient} = 100 \times (FAR_{compound}/FAR_{verapamil})$$

**Inhibition of MDR efflux pumps exerted by the tested compounds.**

FAR and quotient values have been calculated as mentioned above in the methodology of evaluation of the inhibition of the multidrug resistance efflux pumps. To express the cytotoxicity results in Table 3, four intervals have been defined in function of the quotient values determined from FAR values.

- Very potent inhibitors of MDR efflux pumps: Quotient ≥ 200% (≥ 2-fold more potent than reference verapamil). Signalled as ***.
- Potent inhibitors of MDR efflux pumps: 100% ≤ Quotient < 200% (from 1- to 2-fold more potent than reference verapamil). Signalled as **.
- Moderate inhibitors of MDR efflux pumps: 50% ≤ Quotient < 100% (inhibition higher than the 50% of the exerted by the reference verapamil). Signalled as *.
- Not active compounds: Quotient ≤ 50%. Signalled as NI.

*Table 3: Inhibition of MDR efflux pumps exerted by the tested selenoesters in rhodamine assay.*

| Compound | Inhibition of efflux pumps | | Compound | Inhibition of efflux pumps | |
|---|---|---|---|---|---|
| | *At 2 μM concentration* | *At 20 μM concentration* | | *At 2μM concentration* | *At 20 μM concentration* |
| **18** | ** | ** | **22** | *** | *** |
| **19** | ** | ** | **23** | *** | *** |
| **20** | ** | ** | **24** | *** | *** |

**Determination of the minimum inhibitory concentration (MIC) values in different bacterial strains. Methodology.**

The effects exerted by different concentrations of the compounds on bacterial growth in *Staphylococcus aureus 25923 ATCC*, *Staphylococcus aureus multidrug resistant 272123* clinical isolate (MRSA), *Escherichia coli* AG100 or *Escherichia coli* AG100 A were tested in 96-well plates. The MIC of selenocompounds was determined according to Clinical and Laboratory Standard Institute (CLSI) guidelines. The solvent used (DMSO) had no antibacterial effect. Alternatively, the MICs of oxacillin of the compounds in MRSA HEMSA 5 were determined by a broth microdilution method in cation-adjusted Mueller-Hinton Broth (MHB II) according to the recommendations of the CLSI. Results were recorded after a 20- or 24-hour incubation at 37°C.

**Antibacterial activity of the selenocompounds.**

MIC values have been calculated as mentioned above in the methodology of determination of the MIC values in different bacterial strains. To express the antibacterial activity in Table 4, five intervals have been defined in function of the MIC values:

- Very potent antibacterial compounds: MIC ≤ 1 μM. Signalled as ****.
- Potent antibacterial compounds: 1 μM < MIC ≤ 10 μM. Signalled as ***.
- Moderate antibacterial compounds: 10 μM < MIC ≤ 50 μM. Signalled as **.
- Slight antibacterial compounds: 50 μM < MIC ≤ 100 μM. Signalled as *.
- Not antibacterial compounds: MIC ≥ 100 μM. Signalled as NA.

*Table 4: Antibacterial activity of the selenocompounds.*

| Compoun d | Antibacterial activity | | | |
|---|---|---|---|---|
| | *Against S. aureus 25923 ATCC* | *Against S. aureus MRSA 272123* | *Against E. coli AG100* | *Against E. coli AG100 A* |
| **18** | *** | ** | ** | ** |
| **19** | *** | ** | NA | NA |
| **20** | ND | ND | NA | NA |
| **22** | **** | *** | ** | ** |
| **23** | **** | *** | ** | ** |
| **24** | **** | **** | ** | ** |

ND: Not determined.

**Evaluation of the antifungal and nematicidal activity. Methodology.**

The compounds were tested for their antifungal and nematicidal properties against the fungi *Candida albicans* and *Saccharomyces cerevisiae*, and against the nematode *Steinernema feltiae.*

Fungal cell densities were determined by measuring the optical density (OD) at 570 nm at 0 h (first measurement immediately after incubation), 4 h, and 24 h after incubation at 37 °C; and using a Universal Microplate Reader, EL800, Biotech Instruments, Inc. Highland Park, Winooski, VT, USA. Compounds were dissolved in 1% DMSO and were tested at final concentrations of 50, 100, 150, and 200 μM. Nutrient *Sabouraud Dextrose* (SD), and *Yeast Peptone Dextrose* (YPD) were used as media for *C. albicans*, and *S. cerevisiae*, respectively. Ketoconazole was used as the positive control for *C. albicans* and *S. cerevisiae.* The growth culture with medium for each organism was used as the negative control. The Solvent 1% DMSO stock was used as a solvent control. All experiments were carried out in triplicate and on three different occasions (*n* = 9).

The soil nematodes *S. feltiae* were purchased from Sautter and Stepper GmbH (Ammerbuch, Germany) and stored at 4 °C in the dark. Before each experiment, fresh samples of nematodes were purchased and stored in the fridge upon arrival and used for a maximum of six days. To perform the assay, 200 mg of nematodes was reconstituted in 50 mL of phosphate buffered saline (PBS) (pH = 7.4) to form a homogenous suspension to revive the nematodes. Afterwards, the individual suspensions were allowed to rest at room temperature. The sedimentation was avoided with occasional shaking in moderate light and at room temperature (25 °C) for 30 min. Viability of the nematodes was determined by counting the live and dead nematodes under a light microscope (TR 200, VWR International, Leuven, Belgium) at four-fold magnification. An initial viability value of more than 90 percent was considered necessary to perform the experiments.

Sample solutions were added into the wells of a 96-well plate to achieve at final concentrations of 50,100, 150, and 200 μM. Afterwards, 10 μL of nematode suspension was added to each well and the final volume in each well was adjusted to 100 μL with phosphate buffered saline (PBS) (pH = 7.4). PBS was used in the negative control and ethanol in the positive control. Viability was determined at 0 h and 24 h by counting the live and dead nematodes, whereby 50 μL of lukewarm water (40 °C) were added to each well to stimulate the nematodes for the 24 h reading. Each experiment was performed independently in triplicate and on three different occasions (*n* = 9).

For the three organisms considered (*C. albicans, S. cerevisiae* and *S. feltiae*), the inhibitory concentration 50% ($IC_{50}$) is defined as the concentration that inhibits the viability of the respective organism to a 50% of the viability determined for the negative control.

**Antifungal and nematicidal activity of the selenocompounds.**

$IC_{50}$ values have been calculated as mentioned above in the methodology for the evaluation of the antifungal and nematicidal activity. To express the antifungal or nematicidal activity in Table 4, five intervals have been defined in function of the $IC_{50}$ values:

- Very potent antifungal/nematicidal compounds: $IC_{50} \leq 1$ μM. Signalled as ****.
- Potent antifungal/nematicidal compounds: 1 μM < $IC_{50} \leq 10$ μM. Signalled as ***.
- Moderate antifungal/nematicidal compounds: 10 μM < $IC_{50} \leq 50$ μM. Signalled as **.
- Slight antifungal/nematicidal compounds: 50 μM < $IC_{50} \leq 100$ μM. Signalled as *.
- Not antifungal/nematicidal compounds: $IC_{50} \geq 100$ μM. Signalled as NF/NM, respectively.

| Compound | Antifungal activity | | Nematicidal activity |
|---|---|---|---|
| | *Candida albicans* | *Streptomyces cerevisiae* | *Steinernema feltiae* |
| **1** | ** | NF | * |
| **3** | ** | NF | ** |
| **4** | NF | * | ** |
| **5** | ** | NF | *** |
| **6** | ** | NF | ** |
| **7** | ** | NF | *** |

(continued)

| Compound | Antifungal activity | | Nematicidal activity |
|---|---|---|---|
| | *Candida albicans* | *Streptomyces cerevisiae* | *Steinernema feltiae* |
| **8** | NF | * | ** |
| **9** | * | NF | * |
| **10** | ** | NF | NM |
| **11** | ** | NF | * |
| **13** | ** | NF | ** |
| **14** | NF | * | ** |
| **15** | ** | NF | NM |
| **17** | ** | ** | ** |
| **18** | * | NF | ** |
| **19** | ** | ** | ** |
| **20** | ** | ** | NM |
| **22** | ** | * | * |
| **23** | ** | NF | NM |
| **24** | NF | NF | NM |

## Claims

**1.** A compound of formula I for use in the treatment of microbial infections or colorectal cancer:

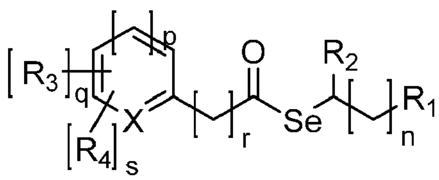

**I**

wherein:

$R_1$ represents -Ph, -CN, -PhR$_5$, -COR$_5$, -SR$_5$, -(SO)R$_5$ or -(SO$_2$)R$_5$;
$R_2$ represents -H, -CH$_3$, -CH$_2$CH$_3$, -Ph or -COCH$_3$;
each $R_3$ independently represents -(CH$_2$)$_r$-COSeCHR$_2$-(CH$_2$)$_n$-R$_1$;
each $R_4$ independently represents -H, -F, -Cl, -Br, -I, -(CH$_2$)$_t$H, -CH(CH$_3$)$_2$,-C(CH$_3$)$_3$, -OH, -OR$_6$, -OCOR$_6$, -SR$_6$, -(SO)R$_6$, -(SO$_2$)R$_6$, -CN, -CF$_3$, -COR$_6$, -COOH,-Ph, -NH$_2$, -NHR$_6$, -N(R$_6$)$_2$, or-NO$_2$;
each $R_5$ independently represents -(CH$_2$)$_t$H, -CH(CH$_3$)$_2$, -C(CH$_3$)$_3$, -CF$_3$, -Ph or-PhR$_4$;
each $R_6$ independently represents -(CH$_2$)$_t$H, -CH(CH$_3$)$_2$, -C(CH$_3$)$_3$ or-Ph;
t represents an integer that ranges between 1 and 6;
X represents CH, N or S;
p represents 0 or 1;
n represents an integer that ranges between 0 and 5;
q represents 0, 1 or 2;
r represents 0, 1 or 2; and
s represents an integer that ranges between 1 and 3,
with the proviso that the following compounds are excluded:

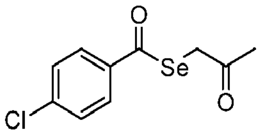

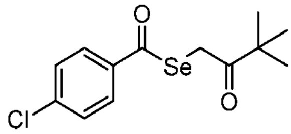

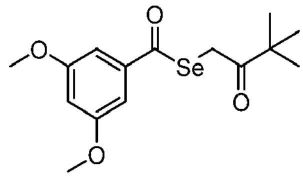

and 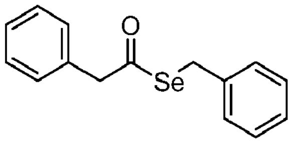 .

2. The compound for use according to claim 1, wherein $R_1$ represents $-PhR_5$,$-COR_5$, $-SR_5$, $-(SO)R_5$ or $-(SO_2)R_5$.

3. The compound for use according to any of claims 1 or 2, , wherein $R_2$ represents -H.

4. The compound for use according to any of claims 1 to 3, wherein each $R_4$ independently represents $-(CH_2)_tH$ or-CN.

5. The compound for use according to any of claims 1 to 4, wherein t represents 1.

6. The compound for use according to any of claims 1 to 5, wherein n represents 0.

7. A compound of formula **II:**

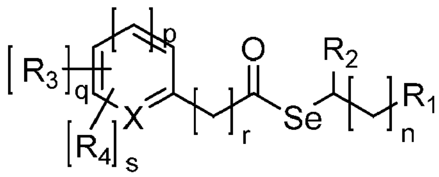

**II**

wherein:

$R_1$ represents -CN, $-PhR_5$, $-SR_5$, $-(SO)R_5$ or $-(SO_2)R_5$;
$R_2$ represents -H, $-CH_3$, $-CH_2CH_3$ or $-COCH_3$;
each $R_3$ independently represents $-[CH_2]_r-COSeCHR_2-[CH_2]_n-R_1$;
each $R_4$ independently represents -H, -F, -Cl, -Br, -I, $-(CH_2)_tH$, $-CH(CH_3)_2$,$-C(CH_3)_3$, -OH, $-OR_7$, $-OCOR_7$, $-SR_7$, $-(SO)R_7$, $-(SO_2)R_7$, -CN, $-CF_3$, $-COR_7$, -COOH, -Ph, $-NH_2$, $-NHR_6$, $-N(R_7)_2$, $-NO_2$;
each $R_5$ independently represents $-(CH_2)_tH$, $-CH(CH_3)_2$, $-C(CH_3)_3$, $-CF_3$, -Ph, $-PhR_4$;
each $R_6$ independently represents $-(CH_2)_tH$, $-CH(CH_3)_2$, $-C(CH_3)_3$, $-CF_3$, $-PhR_8$;
each $R_7$ independently represents $-(CH_2)_tH$, $-CH(CH_3)_2$, $-C(CH_3)_3$, -Ph;
$R_8$ represents -F, -Cl, -Br, -I, $-(CH_2)_tH$, $-CH(CH_3)_2$, $-C(CH_3)_3$, -OH, $-OR_7$, $-SR_7$,$-(SO)R_7$, $-(SO_2)R_7$, -CN, $-CF_3$, $-COR_7$, -COOH, -Ph, $-NH_2$, $-NH(R_6)_2$, $-N(R_7)_2$ or $-NO_2$;
t represents an integer that ranges between 1 and 6;
X represents CH, N or S;
p represents 0 or 1;
n represents an integer that ranges between 0 and 5;
q represents 0, 1 or 2;
r represents 0, 1 or 2; and
s represents an integer that ranges between 1 and 3,
with the proviso that the following compounds are excluded:

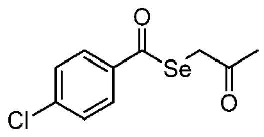
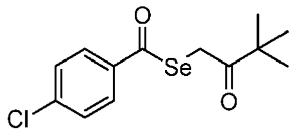
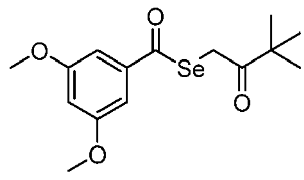
.

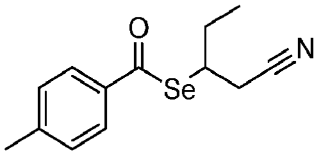
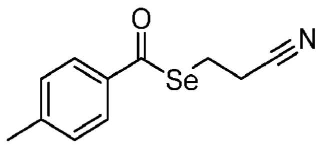
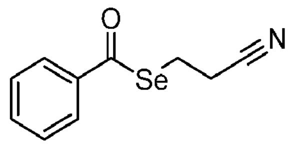

and 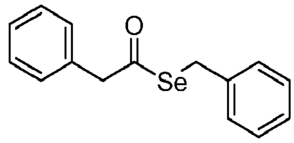.

**8.** The compound of formula **II** according to claim 7, wherein $R_2$ represents -H.

**9.** The compound of formula **II** according to any of claims 7 or 8, wherein each $R_4$ independently represents $-(CH_2)_tH$ or-CN.

**10.** The compound of formula **II** according to any of claims 7 to 9, wherein t represents 1.

**11.** The compound of formula **II** according to any of claims 7 to 10, wherein n represents 0.

**12.** The compound of formula **II** according to claim 7, selected from:

*Se*-(cyanomethyl) 4-fluorobenzoselenoate (**1**);
*Se*-(cyanomethyl) 4-chlorobenzoselenoate (**3**);
*Se*-(cyanomethyl) benzoselenoate (**6**);
*Se*-(cyanomethyl) 4-methylbenzoselenoate (**7**);
*Se*-(cyanomethyl) 2-phenylethaneselenoate (**11**);
*S*e-(cyanomethyl) 2,4-dichlorobenzoselenoate (**13**);
*Se*-(cyanomethyl) 3,4-dichlorobenzoselenoate (**15**);
*Se*-(cyanomethyl) 2-acetoxybenzoselenoate (**17**);
*Se,Se-bis*-(cyanomethyl) benzene-1,4-bis-(carboselenoate) (**18**);
*Se,Se*-bis-(cyanomethyl) benzene-1,3-bis-(carboselenoate) (**19**);
*Se,Se,Se*-tris-(cyanomethyl) benzene-1,3,5-tris-(carboxyselenoate) (**20**);
*Se,Se*-bis-(2-oxopropyl) benzene-1,4-bis-(carboselenoate) (**22**);
*Se,Se*-bis-(2-oxopropyl) benzene-1,3-bis-(carboselenoate) (**23**); and
*Se,Se,Se*-tris-(2-oxopropyl) benzene-1,3,5-tris-(carboxyselenoate) (**24**).

**13.** A pharmaceutical composition which comprises a compound of any of claims 7 to 12 or a pharmaceutical acceptable salt thereof and one or more pharmaceutically acceptable excipients.

**14.** The compound of any of claims 7 to 12, a pharmaceutical acceptable salt thereof or the pharmaceutical composition of claim 13 for use in therapy.

**15.** The compound of any of claims 7 to 12, a pharmaceutical acceptable salt thereof or the pharmaceutical composition of claim 13 for use in the treatment of microbial infections or colorectal cancer.

**16.** The compound of any of claims 7 to 12, a pharmaceutical acceptable salt thereof or the pharmaceutical composition of claim 13 for use in the treatment of microbial infections caused by *Staphylococcus aureus,* methicillin resistant *Staphylococcus aureus, Candida spp* and/or *Escherichia coli.*

Europäisches Patentamt
European Patent Office
Office européen des brevets

# EUROPEAN SEARCH REPORT

Application Number
EP 18 38 2693

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | M. GAJDÁCS, ET AL.: "Selenoesters and selenoanhydrides as novel multidrug resistance reversing agents: A confirmation study in a colon cancer MDR cell line", BIOORGANIC & MEDICINAL CHEMISTRY LETTERS, vol. 27, no. 4, 13 January 2017 (2017-01-13), pages 797-802, XP029906445, Elsevier Science Publishers, Oxford, GB ISSN: 0960-894X, DOI: 10.1016/j.bmcl.2017.01.033 | 1-3,6 | INV. C07C391/00 A61K31/095 A61K31/12 A61K31/277 A61P31/04 A61P35/00 |
| A | * tables 1-4; compounds 2-11 * | 4,5,7-16 | |
| X | H. ISHIHARA, ET AL.: "A facile preparation of aliphatic and aromatic primary selenoamides using 4-methylselenobenzoate as a new selenating reagent", CHEMISTRY LETTERS, vol. 27, no. 12, December 1998 (1998-12), pages 1287-1288, XP055556742, Chemical Society of Japan, Tokyo, JP ISSN: 0366-7022, DOI: 10.1246/cl.1998.1287 * compounds 4a, 4b, 4c, 4d * | 7,9,10 | |
| | -/-- | | TECHNICAL FIELDS SEARCHED (IPC) |
| | | | C07C A61K A61P |

The present search report has been drawn up for all claims

| Place of search | Date of completion of the search | Examiner |
|---|---|---|
| The Hague | 28 February 2019 | English, Russell |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 18 38 2693

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | C.C. TJIN, ET AL.: "Glutathione-responsive selenosulfide prodrugs as a platform strategy for potent and selective mechanism-based inhibition of protein tyrosine phosphatases", ACS CENTRAL SCIENCE, vol. 3, no. 12, 6 December 2017 (2017-12-06), pages 1322-1328, XP055562450, American Chemical Society, Washington, DC, US ISSN: 2374-7943, DOI: 10.1021/acscentsci.7b00486 * compounds 13c, 13d * -& C.C. TJIN, ET AL.: "Supporting information: Glutathione-responsive selenosulfide prodrugs as a platform strategy for potent and selective mechanism-based inhibition of protein tyrosine phosphatases", ACS CENTRAL SCIENCE, vol. 3, no. 12, 6 December 2017 (2017-12-06), pages S1-S58, XP055562454, American Chemical Society, Washington, DC, US ISSN: 2374-7943, DOI: 10.1021/acscentsci.7b00486 ----- | 7,11 | |
| | | | TECHNICAL FIELDS SEARCHED (IPC) |

The present search report has been drawn up for all claims

| Place of search | Date of completion of the search | Examiner |
|---|---|---|
| The Hague | 28 February 2019 | English, Russell |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **GAJDÁCS et al.** *Bioorg Med Chem Lett.,* 2017, vol. 27 (4), 797-802
- **ÁLVAREZ-PÉREZ et al.** *Molecules,* 2018, vol. 23 (3), E628
- **SPENGLER et al.** *Anticancer Res.,* 2011, vol. 31, 3285-3288
- **PAGES et al.** *Curr Med Chem,* 2011, vol. 18, 2969-80
- **DAYAN et al.** *Expert Rev Vaccines,* 2016, vol. 15, 1373-1392
- **BEN-AMI.** *J Fungi,* 2018, vol. 4, E97
- **KUMAR et al.** *Scand J Infect Dis,* 2010, vol. 42, 266-274
- **CIHALOVA et al.** *Int J Mol Sci,* 2015, vol. 16, 24656-24672